(19) Europäisches Patentamt / European Patent Office / Office européen des brevets

(11) **EP 4 289 424 A1**

(12) **EUROPEAN PATENT APPLICATION**
published in accordance with Art. 153(4) EPC


(43) Date of publication:
**13.12.2023 Bulletin 2023/50**

(21) Application number: **22749811.0**

(22) Date of filing: **04.02.2022**

(51) International Patent Classification (IPC):
***A61K 31/40*** *(2006.01)* ***A61K 9/70*** *(2006.01)*
***A61P 25/00*** *(2006.01)*

(52) Cooperative Patent Classification (CPC):
**A61K 9/70; A61K 31/40; A61K 31/60; A61P 1/02; A61P 25/00; C07D 207/12**

(86) International application number:
**PCT/JP2022/004426**

(87) International publication number:
**WO 2022/168940 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.02.2021 JP 2021017091**



(71) Applicant: **SANWA KAGAKU KENKYUSHO CO., LTD.**
**Aichi 461-8631 (JP)**

(72) Inventors:
- **UECHI Kazuhiro**
  **Nagoya-shi, Aichi 461-8631 (JP)**
- **SAKAIRI Masao**
  **Nagoya-shi, Aichi 461-8631 (JP)**
- **SUGITANI Sou**
  **Nagoya-shi, Aichi 461-8631 (JP)**
- **MORIMOTO Nobutaka**
  **Nagoya-shi, Aichi 461-8631 (JP)**
- **TAKADA Kazuki**
  **Nagoya-shi, Aichi 461-8631 (JP)**
- **NAKAMURA Takehiko**
  **Nagoya-shi, Aichi 461-8631 (JP)**

(74) Representative: **Grünecker Patent- und Rechtsanwälte**
**PartG mbB**
**Leopoldstraße 4**
**80802 München (DE)**


(54) **PHARMACEUTICAL COMPRISING GLYCOPYRRONIUM-SALICYLATE**


(57) The present invention pertains to a medicine comprising glycopyrronium salicylate or a solvate thereof, wherein the medicine is used for treating or preventing sialorrhea and the like. The present invention provides: a therapeutic drug that enables the treatment of sialorrhea by affixing; and a novel salt of glycopyrronium that is suitable for a transdermal absorptive preparation.


[Figure 1]

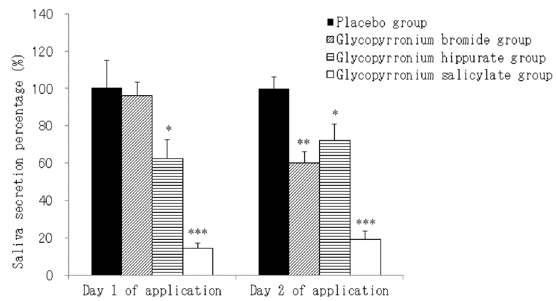


Mean ± S.E.
*:p<0.05, **:p<0.01, ***:p<0.001: Comparison with placebo group (Dunnett's Test)

EP 4 289 424 A1

## Description

TECHNICAL FIELD

The present invention relates to a medicine comprising glycopyrronium salicylate.

BACKGROUND ART

Glycopyrronium is the nonproprietary name for 3-(2-cyclopentyl-2-hydroxy-2-phenylacetoxy)-1,1-dimethylpyrrolidium. Glycopyrronium is a quaternary ammonium cation, and therefore exists as a salt rather than in a free state. For example, a bromide and a tosylate as a glycopyrronium salt are known as an active ingredient of a prescription drug. As another glycopyrronium salt, an iodide, an acetate, a sulfate (for all of them, Patent Literature 1), a chloride, a benzoate, an edisylate, an oxalate (for all of them, Patent Literature 2), and a fatty acid salt (Patent Literature 3) are known.

Glycopyrronium has a muscarinic acetylcholine receptor antagonistic (anticholinergic) action, and an inhalant for chronic obstructive pulmonary diseases (bromide: SEEBRI (registered trademark)), a wet wipe for primary hyperhidrosis (tosylate: QBREXZA (registered trademark)), and an oral solution for sialorrhea (bromide: CUVPOSA (registered trademark)) are approved as a prescription drug in Japan and/or overseas.

Sialorrhea is also called hypersalivation and the symptom thereof is that saliva drips to the outside of the oral cavity. Sialorrhea is considered to lead not only to dripping of saliva but also to difficulty in social life, dermatitis around the mouth, an increased risk of aspiration pneumonia, and the like. Drug treatment is attempted for severe sialorrhea in need of treatment.

Sialorrhea often develops in association with neuromuscular diseases (for example, Parkinson’s disease and amyotrophic lateral sclerosis). Since these patients often have dysphagia as a complication, an external drug (patch) is more desirable than an internal drug for the drug treatment. Advantages of the patch (in particular, transdermal absorptive preparation) include easy administration to a patient with swallowing difficulty, expectation of reduction of side effects due to reduction of exposure amount of the drug, simple administration, easy medication management, and easy discontinuation of administration.

Pharmaceutical products are classified, according to the administration route, into an "internal drug", an "injection", and an "external drug". The external drug is a medicine for causing a drug to be absorbed from the skin or mucous membrane, other than the internal drug and the injection. Specific external drugs for causing a drug to be absorbed from the skin include a patch, an ointment, and a wet wipe. The patch is classified, according to the site of action, into a transdermal absorptive preparation and a topical preparation. The transdermal absorptive preparation is defined as a preparation which exhibits drug efficacy by circulation of the drug absorbed from the skin in the systemic blood flow. On the other hand, the topical preparation is defined as a preparation that exhibits drug efficacy by increase of the drug concentration in the target tissue near the application site without significant increase of the blood concentration of the drug absorbed from the skin.

As an external drug of glycopyrronium, a transdermal drug delivery system for treating sialorrhea containing a glycopyrronium bromide (Patent Literature 4) and a topical composition containing a glycopyrronium tosylate (Patent Literature 2) are known. Since Patent Literature 4 does not describe Examples and does not describe drug efficacy in animals or humans, either, the feasibility of the transdermal drug delivery system remains questionable, and, additionally, it is not clear whether the topical composition can be referred to as a transdermal absorptive preparation. Patent Literature 2 describes a wet wipe as a specific example of the topical composition. It is an external drug, but is not a transdermal absorptive preparation, and is applied and used. Thus, strict control of the dose and keeping constant drug absorption cannot be expected, and it is difficult to achieve a continuously stable amount of drug exposure in the blood.

For a transdermal absorptive preparation containing a drug, it is necessary for the drug to have high skin permeability. In general, the amount of the absorbed drug at the time of transdermal administration is determined based on the administration area and the skin permeability of the drug. Since it is necessary to avoid repeated administration to the same site in consideration of safety to the skin, it is restricted to achieve a sufficient amount of the absorbed drug even by expanding the administration area. A transdermal absorptive preparation containing a bromide or tosylate of glycopyrronium which is intended to achieve a continuously stable amount of drug exposure in the blood is not commercially available not only in Japan but also overseas.

CITATIONS LIST

PATENT LITERATURE

Patent Literature 1: JP 2008-534480 T
Patent Literature 2: JP 2017-128593 A
Patent Literature 3: JP 2018-519289 T
Patent Literature 4: JP 2010-530902 T

## SUMMARY OF INVENTION

### TECHNICAL PROBLEMS

For the treatment or prevention of sialorrhea, an external drug, particularly a transdermal absorptive preparation, is more preferable than an internal drug. An internal drug, an external drug (inhalant), and an injection are commercially available for glycopyrronium bromide, and an external drug (wet wipe) is commercially available for glycopyrronium tosylate, but transdermal absorptive preparations are not commercially available for either salt. From such a background, an object of the present invention is to provide a medicine (transdermal absorptive preparation) that enables the treatment or prevention of sialorrhea by affixing, and further to provide a novel salt of glycopyrronium suitable for a transdermal absorptive preparation.

### SOLUTIONS TO PROBLEMS

The present inventors considered that the reason why a medicine for the treatment or prevention of sialorrhea by affixing, that is, a transdermal absorptive preparation, has not been provided, is due to the active ingredient, i.e., a glycopyrronium bromide or tosylate. As a result of intensive studies, the present inventors have found that, it is necessary to select glycopyrronium salicylate as an active ingredient, in order to provide a medicine that enables the treatment or prevention of sialorrhea through being affixed, and have completed the present invention.
Main configurations of the present invention are as follows.

[1] A medicine comprising glycopyrronium salicylate or a solvate thereof, wherein the medicine is used for treating or preventing sialorrhea.
[2] The medicine according to [1], wherein the medicine is used so as to be affixed.
[3] The medicine according to [2], wherein the medicine is used so as to be affixed once a day.
[4] A medicine comprising glycopyrronium salicylate or a solvate thereof, wherein the medicine is used so as to be affixed once a day and be re-affixed every 24 hours for treating or preventing sialorrhea.
[5] The medicine according to [1] to [4], wherein the medicine is a transdermal absorptive preparation.
[6] Glycopyrronium salicylate or a solvate thereof.
[7] A medicine comprising glycopyrronium salicylate or a solvate thereof.
[8] A transdermal absorptive preparation comprising a plaster layer and a release liner on a backing, wherein the plaster layer comprises an active ingredient and the active ingredient is glycopyrronium salicylate.
[9] Glycopyrronium salicylate or a solvate thereof for treating or preventing sialorrhea.
[10] Use of glycopyrronium salicylate or a solvate thereof for the manufacture of a medicine for treating or preventing sialorrhea.
[11] A method for treating or preventing sialorrhea by administering glycopyrronium salicylate or a solvate thereof to a patient in need thereof.

### ADVANTAGEOUS EFFECTS OF INVENTION

The present invention can provide a medicine which comprises glycopyrronium salicylate as an active ingredient and, upon being affixed, exhibits an excellent therapeutic or preventive effect on sialorrhea, that is, a transdermal absorptive preparation.

## BRIEF DESCRIPTION OF DRAWINGS

Fig. 1 is a figure showing an effect of transdermal absorptive preparations (0.5 $cm^2$) containing various glycopyrronium salts on salivary secretion in a rat pilocarpine-induced salivary secretion model.
Fig. 2 is a figure showing an effect of transdermal absorptive preparations (0.25 $cm^2$) containing various glycopyrronium salts on salivary secretion in a rat pilocarpine-induced salivary secretion model.

DESCRIPTION OF EMBODIMENT

Hereinafter, a medicine for the treatment or prevention of sialorrhea according to the present invention will be described.

The medicine for the treatment or prevention of sialorrhea according to the present invention is usually a pharmaceutical composition for the treatment or prevention of sialorrhea.

An active ingredient for use in the medicine for the treatment or prevention of sialorrhea according to the present invention is 3-(2-cyclopentyl-2-hydroxy-2-phenylacetoxy)-1,1-dimethylpyrrolidium salicylate (hereinafter, referred to as "glycopyrronium salicylate") represented by Formula (1) below. Unless otherwise specified herein, the present invention also encompasses a solvate of glycopyrronium salicylate. In addition, in the present specification, the term glycopyrronium salicylate may include a solvate thereof. In addition, glycopyrronium has four stereoisomers, i.e., (3R, 2R), (3R, 2S), (3S, 2R), and (3S, 2S), from its structure. The glycopyrronium salicylate as the active ingredient of the present invention may be a mixture containing several stereoisomers, but a mixture of (3R, 2S) and (3S, 2R) in combination is preferable, and (3S, 2R) stereoisomer alone is preferable.

[Chemical Formula 1]

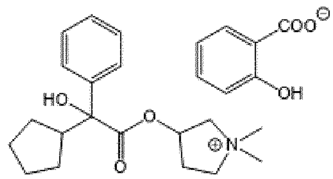

(1)

The compound of the present invention can be produced by a method as shown in Reaction Step Formulas 1 and 2 below or a method obtained by combining known methods.

[Reaction Step Formula 1]

[Chemical Formula 2]

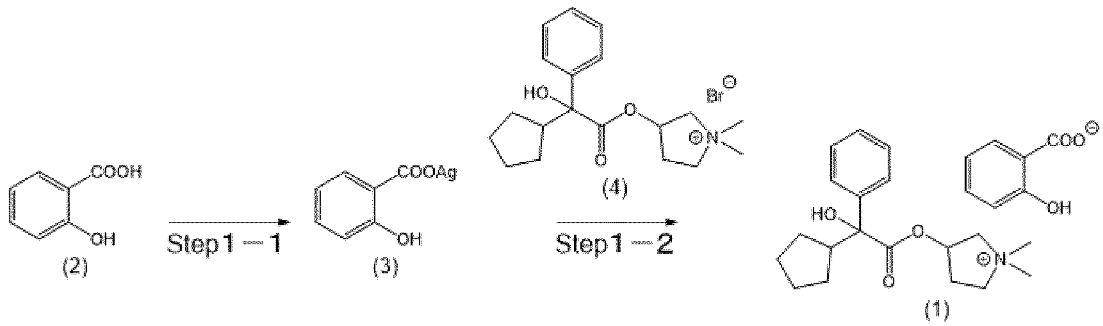


wherein R represents a hydrogen atom or an alkali metal atom.

[Step 1-1]

Silver salicylate (3) can be obtained by reacting salicylic acid (2) with a silver salt (for example, silver oxide, silver nitrate, or the like) in a suitable solvent (for example, distilled water, acetone, acetonitrile, or the like, or a mixed solvent thereof), usually at a temperature of from 0°C to the boiling point of the solvent, for a period of from 10 minutes to 3 days in a light-shielded state.

[Step 1-2]

Glycopyrronium salicylate (1) can be obtained by reacting glycopyrronium bromide (4) with the silver salicylate

produced in Step 1-1 above in an appropriate solvent (for example, distilled water, acetone, acetonitrile, or the like, or a mixed solvent thereof), usually at a temperature of from 0°C to the boiling point of the solvent, for a period of from 1 hour to 3 days in a light-shielded state; and filtering the precipitated silver bromide.

[Reaction Step Formula 2]

[Chemical Formula 3]

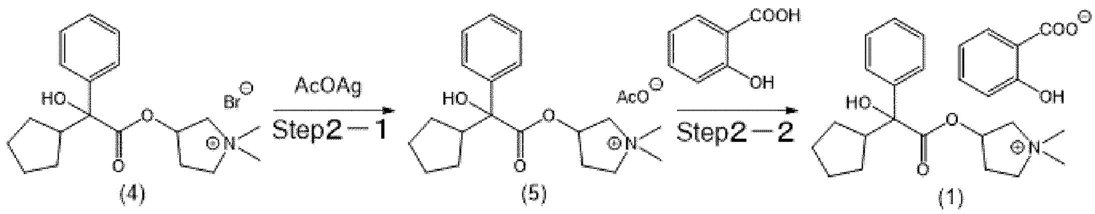


[Step 2-1]

Glycopyrronium acetate (5) can be obtained by reacting the glycopyrronium bromide (4) with silver acetate in an appropriate solvent (for example, distilled water, acetone, acetonitrile, or the like, or a mixed solvent thereof), usually at a temperature of from 0°C to the boiling point of the solvent, for a period of from 1 hour to 3 days in a light-shielded state; and filtering the precipitated silver bromide.

[Step 2-2]

Glycopyrronium salicylate (1) can be obtained by reacting the glycopyrronium acetate (5) produced in Step 2-1 above with salicylic acid in an appropriate solvent (for example, distilled water, acetone, acetonitrile, or the like, or a mixed solvent thereof), usually at a temperature of from 0°C to the boiling point of the solvent, for a period of from 1 hour to 3 days.

The medicine for the treatment or prevention of sialorrhea according to the present invention is not limited, but is usually a patch including a backing, a plaster layer, and a release sheet, and is preferably a transdermal absorptive preparation. The plaster layer contains a pressure-sensitive adhesive and glycopyrronium salicylate, and is usually layered on a backing or a release sheet.

The pressure-sensitive adhesive is not particularly limited as long as it can be used as a patch. There is a difference in pressure-sensitive adhesive strength depending on the type of pressure-sensitive adhesive, but the type of pressure-sensitive adhesive and the blending amount thereof can be appropriately selected according to the desired application time. Examples of the pressure-sensitive adhesive include an acrylic pressure-sensitive adhesive, a rubber-based pressure-sensitive adhesive, a silicon-based pressure-sensitive adhesive, and the like.

Specific examples of the acrylic pressure-sensitive adhesive include an acrylic acid-octyl acrylate copolymer, an acrylic acid ester-vinyl acetate copolymer, an ethyl acrylate-octyl acrylate-vinyl pyrrolidone copolymer, a 2-ethylhexyl acrylate-vinyl acetate copolymer, a 2-ethylhexyl acrylate-hydroxyethyl acrylate-vinyl acetate-glycidyl methacrylate copolymer, a 2-ethylhexyl acrylate-vinyl pyrrolidone copolymer, a 2-ethylhexyl acrylate-methyl acrylate-acrylic acid-glycidyl methacrylate copolymer, a 2-ethylhexyl acrylate-vinyl acetate-hydroxyethyl acrylate-glycidyl methacrylate copolymer, a 2-ethylhexyl acrylate-vinyl acetate-butyl acrylate-acrylic acid copolymer, a 2-ethylhexyl acrylate-vinyl acetate-acrylic acid copolymer, a 2-ethylhexyl acrylate-diacetone acrylamide-acetoacetoxyethyl methacrylate-methyl methacrylate copolymer, a 2-ethylhexyl acrylate-2-ethylhexyl methacrylate-dodecyl methacrylate copolymer, an aminoalkyl methacrylate copolymer E, and the like.

Specific examples of the rubber-based pressure-sensitive adhesive include a styrene-isoprene-styrene copolymer, an isoprene rubber, polyisobutylene, a styrene-butadiene-styrene block copolymer, a styrene-butadiene rubber, and the like.

Specific examples of the silicon-based pressure-sensitive adhesive include dimethylpolysiloxane, a dimethylpolysiloxane-silicate resin condensation product, and the like.

In the medicine for the treatment or prevention of sialorrhea according to the present invention, additives suitable for various purposes can be further added to the plaster layer. Such additives are not particularly limited, and examples thereof include absorption promoters, stabilizers, solubilizers, buffers, antioxidants, fragrances, cooling agents, flavoring

agents, colorants, tackiness enhancers, pH adjusters, excipients, extenders, preservatives, dissolvers, dissolving aids, and other medically acceptable additives.

The medicine for the treatment or prevention of sialorrhea according to the present invention is intended to allow a drug to be continuously and stably absorbed from the skin, and is different from a topical action type medicine in that a stable exposure amount of the drug in the blood can be obtained and that the medicine is continuously affixed.

A method for manufacturing the medicine for the treatment or prevention of sialorrhea according to the present invention includes, for example; (I) a method for manufacturing the medicine by dissolving glycopyrronium salicylate, a pressure-sensitive adhesive, and other additives in an organic solvent such as ethyl acetate, ethanol, methanol, hexane, or toluene, a mixed solvent thereof, applying the solution onto one of a release sheet or a backing to evaporate the solvent, thereby forming a drug-containing layer, and then bonding thereto the other of the release sheet or the backing (on which the drug-containing layer is not formed), (II) a method for manufacturing the medicine by heating and melting glycopyrronium salicylate, a pressure-sensitive adhesive, and other additives, applying the melt onto one of a release sheet or a backing to form a drug-containing layer, and then bonding thereto the other of the release sheet or the backing (on which the drug-containing layer is not formed), and the like. An order of applying the drug mixture in the manufacture method (I) and the melt in the manufacture method (II) onto the backing or the release sheet is not limited. The release sheet may be bonded after a plaster is applied onto the backing first, or the backing may be bonded after the plaster is applied onto the release sheet first.

The backing is not particularly limited as long as it can support the plaster layer, and a stretchable or non-stretchable backing can be used. Specific examples of the backing include a woven fabric, a nonwoven fabric, a vinyl chloride film, a polyethylene film, a polypropylene film, a polyester film, a polyurethane film, composite materials thereof, and the like. A thickness of the backing is not particularly limited, and can be appropriately determined.

The release sheet that can be used is not particularly limited as long as it covers the plaster layer, and a vinyl chloride film, a polyethylene film, a polypropylene film, a polyester film, a polyurethane film, or the like can be used. For the purpose of facilitating peeling of the release sheet, a sheet subjected to a silicone treatment or an embossed sheet can also be used.

## EXAMPLES

Hereinafter, the contents of the present invention will be described in more detail with reference to Examples, but the technical scope of the present invention is not limited to the contents described therein.

The nuclear magnetic resonance spectrum and the melting point were measured under the following measurement conditions.

### [Nuclear magnetic resonance spectrum]

Nuclear magnetic resonance ($^1H$-NMR) spectrum measurement in the following salt production examples and comparative salt production examples was performed using (MR1008W041) manufactured by VARIAN. For the spectrum, a chemical shift value was described as a $\delta$ value (ppm) using tetramethylsilane as a standard substance. The splitting pattern is indicated by "s" for singlet, "d" for doublet, "t" for triplet, and "m" for multiplet.

### [Melting point]

Melting point measurement in the following salt production examples and comparative salt production examples was performed using (Thermo plus TG 8120) manufactured by Rigaku. The measurement conditions were a measurement temperature of from room temperature to 200°C, a heating rate of 5°C/min, and a measurement interval of 0.2 seconds.

### <<Preparation of glycopyrronium salts>>

Six counter anions (carboxylic acids) suitable for pharmaceutical products were selected to attempt the synthesis of the glycopyrronium salts. The selected carboxylic acids are shown in Table 1. As a raw material, a commercially available (3RS, 2SR)-glycopyrronium bromide was used.

[Table 1]

| | |
|---|---|
| 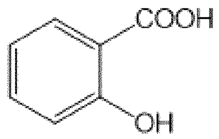 Salicylic acid | 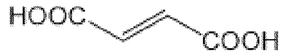 Fumaric acid |
| 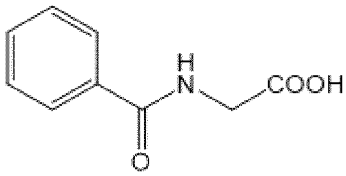 Hippuric acid | 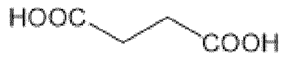 Succinic acd |
| 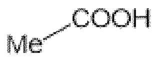 Acetic acid | 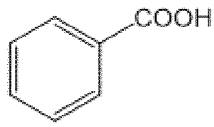 Benzoic acid |

Preparation of glycopyrronium salicylate

[Salt Production Example 1]

A brown eggplant flask was charged with 13 mL of distilled water and 500.0 mg (1.26 mmol) of (3RS, 2SR)-glycopyrronium bromide for dissolution. To the solution, 209.5 mg (1.26 mmol) of silver acetate was added at 40°C in a light-shielded state, and the mixture was stirred for 24 hours. The reaction solution was filtered through Celite and washed with distilled water, and then the filtrate was concentrated under reduced pressure. To the obtained crude glycopyrronium acetate, 10 mL of acetonitrile and 173.5 mg (1.26 mmol) of salicylic acid were added, and the mixture was stirred at room temperature for 18 hours. The reaction solution was concentrated under reduced pressure, and subjected to toluene azeotropy, thereby obtaining a crude product of glycopyrronium salicylate. Toluene (10 mL) was added to the crude product, and the mixture was stirred at room temperature for 5 hours. The suspension in which crystals were precipitated was allowed to stand at -20°C for 9 days, and then the crystals were collected by filtration. The crystals were dried at 40°C under reduced pressure to obtain 517.8 mg of (3RS, 2SR)-glycopyrronium salicylate (yield: 91%).

[$^1$H-NMR] (400 MHz, DMSO-$d_6$) δ 7.63 (ddd, 1H), 7.58 (d, 2H), 7.35 (t, 2H), 7.27 (t, 1H), 7.10 (ddd, 1H), 6.59-6.53 (m, 2H), 5.85 (s, 1H), 5.39-5.35 (m, 1H), 3.83 (dd, 1H), 3.72-3.65 (m, 1H), 3.60 (d, 1H), 3.54-3.47 (m, 1H), 3.15 (s, 3H), 3.08 (s, 3H), 2.95-2.87 (m, 1H), 2.68-2.59 (m, 1H), 2.11-2.03 (m, 1H), 1.65-1.13 (m, 8H).
mp 141°C.

[Salt Production Example 2]

A brown eggplant flask was charged with 5.30 g (31.2 mmol) of silver nitrate and 6.3 mL of distilled water for suspension. 6.3 mL of an aqueous solution containing 5.00 g (31.2 mmol) of sodium salicylate was added thereto, and then the mixture was stirred at room temperature for 15 minutes in a light-shielded state. The precipitated solid was collected by filtration with a Kiriyama funnel, washed with distilled water and ethanol in this order, and then dried under reduced pressure to obtain 6.50 g of silver salicylate (yield: 85%).

A brown eggplant flask was charged with 1.50 g (3.77 mmol) of (3RS, 2SR)-glycopyrronium bromide, 923 mg (3.77 mmol) of silver salicylate and 35 mL of distilled water, and the mixture was stirred at 40°C for 20 hours in a light-shielded state. The reaction solution was filtered through Celite and washed with distilled water, and then the filtrate was concentrated under reduced pressure and subjected to toluene azeotropy, thereby obtaining a crude product of

glycopyrronium salicylate. Toluene (10 mL) was added, and a small amount of solid was precipitated by applying ultrasonic waves. Then, 20 mL of toluene was added, and the mixture was stirred at room temperature overnight. The precipitated crystals were collected by filtration, washed with toluene, and dried at 50°C under reduced pressure to obtain 1.70 g of (3RS, 2SR)-glycopyrronium salicylate (yield: 99%).

Preparation of other glycopyrronium salts

[Comparative Salt Production Example 1]

A brown eggplant flask was charged with 20.0 g (111 mmol) of hippuric acid, 12.9 g (111 mmol) of silver oxide, 440 mL of acetone, and 220 mL of distilled water for suspension, and the mixture was stirred at room temperature for 15 hours in a light-shielded state. The precipitated solid was collected by filtration with a Kiriyama funnel, washed with distilled water, and then dried under reduced pressure to obtain 29.2 g of silver hippurate (yield: 91%).

A brown eggplant flask was charged with 5.00 g (12.6 mmol) of (3RS, 2SR)-glycopyrronium bromide, 3.61 g (12.6 mmol) of silver hippurate, and 100 mL of distilled water, and the mixture was stirred at 40°C for 12 hours in a light-shielded state. The reaction solution was filtered through Celite and washed with distilled water, and then the filtrate was concentrated under reduced pressure and subjected to toluene azeotropy, thereby obtaining a crude product of glycopyrronium hippurate. Ethyl acetate (100 mL) was added thereto, and a small amount of solid was precipitated by applying ultrasonic waves. Thereafter, the suspension was allowed to stand at -20°C for 3 days, and crystals were collected by filtration, washed with ethyl acetate, and then dried at room temperature under reduced pressure to obtain 5.10 g of (3RS, 2SR)-glycopyrronium hippurate (yield: 81%).

[$^1$H-NMR] (400 MHz, DMSO-$d_6$) δ 7.81-7.76 (m, 2H), 7.73-7.67 (m, 1H), 7.58 (d, 2H), 7.53-7.42 (m, 3H), 7.35 (t, 2H), 7.27 (t, 1H), 5.92 (s, 1H), 5.41-5.34 (m, 1H), 3.83 (dd, 1H), 3.72-3.65 (m, 1H), 3.60 (d, 1H), 3.54-3.46 (m, 1H), 3.41 (d, 2H), 3.15 (s, 3H), 3.08 (s, 3H), 2.95-2.85 (m, 1H), 2.69-2.59 (m, 1H), 2.12-2.03 (m, 1H), 1.65-1.13 (m, 8H). mp 135°C.

[Comparative Salt Production Examples 2 and 3]

Glycopyrronium salts shown in Table 2 were synthesized from (3RS, 2SR)-glycopyrronium bromide using the same procedures as in Salt Production Example 1. Table 2 also indicates whether crystals could be generated or not. × represents that no crystal could be obtained. Instrument analysis data on the obtained salts is shown in Table 3.

[Table 2]

| | Structure | Availability of crystal formation |
|---|---|---|
| Comparative Salt Production Example 2 | 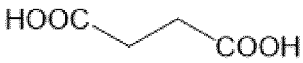 Succinic acd | × |
| Comparative Salt Production Example 3 | 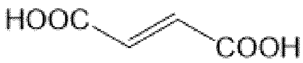 Fumaric acid | × |

[Table 3]

| | |
|---|---|
| Comparative Salt Production Example 2 | $^1$H-NMR (400 MHz, DMSO-d6) δ 7. 59 (d, 2H) . 7. 35 (t, 2H), 7.27 (t, 1H), 5. 83 (s, 1H), 5. 41-5. 34 (m, 1H), 3. 81 (dd, 1H), 3. 71-3. 64 (m, 1H), 3.59 (d, 1H), 3.49 (dt, 1H), 3.14 (s, 3H), 3.08 (s, 3H), 2. 96-2. 87 (m, 1H), 2.68-2. 58 (m, 1H), 2.22 (s, 4H), 2.12-2.02 (m, 1H), 1. 65-1. 45 (m, 5H), 1. 43-1. 33 (m, 1H), 1. 29-1. 12 (m, 2H). |

(continued)

| | |
|---|---|
| Comparative Salt Product ion Example 3 | $^1$H-NMR (400 MHz, DMSO-d6) δ 7. 59 (d, 2H), 7.35 (t, 2H), 7.27 Ct, 1H), 6. 32 (s, 2H), 5.86 (s, 1H), 5.41-5.34 (m, 1H), 3. 82 (dd, 1H), 3.72-3.64 (m, 1H), 3.59 (d, 1H), 3.50 (dt, 1H), 3.14 (s, 3H), 3. 08 (s, 3H), 2. 96-2. 87 (m, 1H), 2. 68-2. 59 (m, 1H), 2. 12-2. 02 (m, 1H), 1. 65-1. 46 (m, 5H), 1. 43-1. 34 (m, 1H), 1. 29-1. 12 (m, 2H). |

[Comparative Salt Production Examples 4 and 5]

Glycopyrronium acetate and glycopyrronium benzoate were synthesized. The acetate was produced with reference to Example 2 of JP 2008-534480 T, and the benzoate was produced with reference to Example 2 of JP 2016-510037 T from (3RS, 2SR)-glycopyrronium bromide. For any of these salts, crystals having a steric structure (3RS, 2SR) could be obtained. The analysis data are omitted.

[Comparative Salt Example 1]

As the glycopyrronium bromide, a commercially available product whose steric structure is specified as (3RS, 2SR) was purchased and used. This is a crystal.

As described above, various glycopyrronium salts were synthesized. No crystals could be obtained for the succinate and fumarate of glycopyrronium, whereas crystals could be obtained for the salicylate, hippurate, acetate, and benzoate of glycopyrronium. When used as an active ingredient of a pharmaceutical product, a solid (particularly, a crystalline) glycopyrronium salt is preferable because it is superior in handleability. With respect to the salicylate, hippurate, acetate, and benzoate of glycopyrronium for which crystals could be obtained, small transdermal absorptive preparations were prepared using the products of above-mentioned Salt Production Examples and Comparative Salt Production Examples (Salt Production Example 1 for glycopyrronium salicylate), and an in vitro skin permeability test was performed in Test Example 1.

As a result of dissolving the four glycopyrronium salts in ethanol used in the production of the transdermal absorptive preparations and evaluating the stability after 24 hours, 20% and 10% decompositions were observed for the acetate and the benzoate, respectively, and 1% or less decompositions were observed for both of the hippurate and the salicylate. With respect to the hippurate and the salicylate of glycopyrronium which were stable in ethanol, and the glycopyrronium bromide existing as the prior art, small transdermal absorptive preparations were produced, and the effectiveness of the three glycopyrronium salts was evaluated in a salivary secretion model animal (Test Examples 2 and 3).

<<Preparation of transdermal absorptive preparation containing glycopyrronium salt>>

[Preparation Production Example 1]

<u>Transdermal absorptive preparation containing glycopyrronium salicylate (6.5% in terms of glycopyrronium)</u>

A mixed solution of Glycopyrronium salicylate and a 2-ethylhexyl acrylate-vinyl acetate-hydroxyethyl acrylate-glycidyl methacrylate copolymer in ethyl acetate-ethanol-heptane-methanol was dissolved in ethanol at a blending ratio shown in Table 4. The obtained solution was applied onto a PET backing such that the amount of the plaster after drying was 15 mg/cm$^2$ (0.98 mg/cm$^2$ in terms of glycopyrronium), and then dried to remove ethanol and the organic solvents contained in the pressure-sensitive adhesive, thereby obtaining a plaster. Next, a PET release sheet was bonded onto the plaster, and then cut into a desired size to obtain a transdermal absorptive preparation.

[Comparative Preparation Production Example 1]

<u>Transdermal absorptive preparation containing glycopyrronium benzoate (6.5% in terms of glycopyrronium)</u>

In the same manner as in Preparation Production Example 1, a transdermal absorptive preparation containing glycopyrronium benzoate at a blending ratio shown in Table 4 was produced.

[Comparative Preparation Production Example 2]

Transdermal absorptive preparation containing glycopyrronium hippurate (6.5% in terms of glycopyrronium)

In the same manner as in Preparation Production Example 1, a transdermal absorptive preparation containing glycopyrronium hippurate at a blending ratio shown in Table 4 was produced.

[Comparative Preparation Production Example 3]

Transdermal absorptive preparation containing glycopyrronium acetate (6.5% in terms of glycopyrronium)

In the same manner as in Preparation Production Example 1, a transdermal absorptive preparation containing glycopyrronium acetate at a blending ratio shown in Table 4 was produced.

[Comparative Preparation Production Example 4]

Transdermal absorptive preparation containing glycopyrronium bromide (6.5% in terms of glycopyrronium)

In the same manner as in Preparation Production Example 1, a transdermal absorptive preparation containing glycopyrronium bromide at a blending ratio shown in Table 4 was produced.

[Reference Preparation Production Example 1]

Placebo patch drug

A mixed solution of a 2-ethylhexyl acrylate-vinyl acetate-hydroxyethyl acrylate-glycidyl methacrylate copolymer in ethyl acetate-ethanol-heptane-methanol was dissolved in ethanol. The obtained solution was applied onto a PET backing such that the amount of the plaster after drying was 15 mg/cm$^2$, and then dried to remove ethanol and the organic solvents contained in the pressure-sensitive adhesive, thereby obtaining a plaster. Next, a PET release sheet was bonded onto the plaster, and then cut into a desired size to obtain a patch drug.

[Table 4]

| Component name | Preparation Production Example 1 (w/w%) | Comparative Preparation Production Example 1 (w/w%) | Comparative Preparation Production Example 2 (w/w%) |
|---|---|---|---|
| Glycopyrronium salicylate | 9. 3 | - | - |
| Glycopyrronium benzoate | - | 9. 0 | - |
| Glycopyrronium hippurate | - | - | 10.1 |
| 2-Ethylhexyl acrylate-vinyl acetate-hydroxyethyl acrylate-glycidyl methacrylate copolymer ethyl acetate-ethanol-heptane-methanol mixed solution | 90.7 | 91 | 89.9 |
| Total | 100 | 100 | 100 |
| Component name | Comparative Preparation Production Example 3 (w/w%) | Comparative Preparation Production Example 4 (w/w%) | Reference Preparation Production Example 1 (w/w%) |
| Glycopyrronium acetate | 7. 7 | - | - |
| Glycopyrronium bromide | - | 8. 1 | - |

(continued)

| Component name | Comparative Preparation Production Example 3 (w/w%) | Comparative Preparation Production Example 4 (w/w%) | Reference Preparation Production Example 1 (w/w%) |
|---|---|---|---|
| 2-Ethylhexyl acrylate-vinyl acetate-hydroxyethyl acrylate-glycidyl methacrylate copolymer ethyl acetate-ethanol-heptane-methanol mixed solution | 92.3 | 91.9 | 100 |
| Total | 100 | 100 | 100 |

<<Skin permeability test>>

[Test Example 1]

In vitro skin permeability test of transdermal absorptive preparations containing various glycopyrronium salts (6.5% in terms of glycopyrronium)

Test method

Using the skins of nude mice (BALB/cSlc-nu/nu, male, 8 to 12 weeks old, Japan SLC, Inc.), the following transdermal absorptive preparations produced in accordance with Preparation Production Example 1 and Comparative Preparation Production Examples 1 to 3 were evaluated for the in vitro skin permeability of glycopyrronium.

. Glycopyrronium salicylate group (n = 3)
. Glycopyrronium benzoate group (n = 3)
. Glycopyrronium hippurate group (n = 3)
. Glycopyrronium acetate group (n = 3)

The transdermal absorptive preparations were applied to the collected skins of the nude mice, and the amounts of the glycopyrronium permeated into the receptor solutions were evaluated using a vertical diffusion cell (opening inner diameter: 15 mm, area: 1.767 $cm^2$, receptor capacity: 9.5 mL, cell temperature: set at 32°C). The transdermal absorptive preparations used had a sufficient size to cover the opening. PBS was used as a receptor solution, and stirred with a magnetic stirrer during the permeation test.

After a certain period of time, the receptor solutions were collected, and the glycopyrronium concentrations were measured using LC-MS/MS. The amounts of the glycopyrronium permeated per unit area were calculated from the glycopyrronium concentrations in the receptor solutions, the receptor capacity, and the opening area.

Results

The results of the amounts of glycopyrronium permeated per unit area 10 hours after the start of permeation of respective transdermal absorptive preparations are shown in Table 5.

The amount of glycopyrronium salicylate which permeated through the skin was about 3 times higher than that of the benzoate and hippurate of glycopyrronium and about 8 times higher than that of glycopyrronium acetate. Among the four groups, the glycopyrronium salicylate group showed the highest in vitro skin permeability. It was revealed that there was a large difference in the amounts of glycopyrronium salts which permeated through the skin depending on the type of glycopyrronium salts.

[Table 5]

| Component name | permeation amount during 10h (ng/$cm^2$) |
|---|---|
| Glycopyrronium salicylate | 2420 ± 580 |
| Glycopyrronium benzoate | 811±168 |
| Glycopyrronium hippurate | 814±202 |

(continued)

| Component name | permeation amount during 10h (ng/$cm^2$) |
|---|---|
| Glycopyrronium acetate | 301±88 |

Mean ± SD

<<Drug Efficacy Test>>

[Test Example 2]

Effect of application of 0.5 $cm^2$ of transdermal absorptive preparations containing various glycopyrronium salts (6.5% in terms of glycopyrronium) on pilocarpine-induced salivary secretion

Test method

Hairless rats (HWY/Slc, male, 8 weeks old, Japan SLC, Inc.) were divided into four groups as follows to perform the test.

. Placebo group (n = 5)
. Glycopyrronium bromide group (n = 5)
. Glycopyrronium hippurate group (n = 5)
. Glycopyrronium salicylate group (n = 5)

Under isoflurane anesthesia, 0.5 $cm^2$ of each of the transdermal absorptive preparations (including a placebo preparation) containing various glycopyrronium salts produced in accordance with Preparation Production Example 1, Comparative Preparation Production Examples 2 and 4, and Reference Preparation Production Example 1 was applied to the necks of the rats once a day every 24 hours for 2 days.

On Day 1 and Day 2 of application (23 and 47 hours after the first application), the rats were administered with 0.5 mg/kg of pilocarpine hydrochloride through the tail vein under anesthesia with a mixture of three anesthetics. A cotton ball was inserted into the oral cavity to collect saliva for 60 minutes. The amount of saliva secreted was calculated from the difference between the weights of the cotton ball before and after collection of saliva, and taking the average value of the amount of saliva secreted of the placebo group as 100%, the salivary secretion proportion of each individual was calculated from the following (equation).

(Equation)

Salivary secretion proportion (%) = (amount of saliva secreted of each individual/average amount of saliva secreted of placebo group) × 100

Results

Fig. 1 shows the results of the salivary secretion proportion for the transdermal absorptive preparation containing each of the glycopyrronium salts on Day 1 and Day 2 of application.

On Day 1 of application, significant salivary secretion suppressive effects were observed in the glycopyrronium hippurate group and the glycopyrronium salicylate group as compared with the placebo group, but no significant effect was observed in the glycopyrronium-bromide group. On Day 2 of application, significant salivary secretion suppressive effects were observed in all the glycopyrronium salt groups as compared with the placebo group, and the glycopyrronium salicylate group showed the strongest salivary secretion suppressive effect.

[Test Example 3]

Effect of application of 0.25 $cm^2$ of transdermal absorptive preparations containing various glycopyrronium salts (6.5% in terms of glycopyrronium) on pilocarpine-induced salivary secretion

Test method

Test Example 3 was performed in the same manner as in Test Example 2 except that the area of the patch drug was changed to 0.25 $cm^2$.

Results

Fig. 2 shows the results of the salivary secretion proportion for the transdermal absorptive preparation containing each of the glycopyrronium salts on Day 1 and Day 2 of application.

On Day 1 and Day 2 of application, a salivary secretion suppression tendencies were observed in all of the glycopyrronium salt groups as compared with the placebo group, but the glycopyrronium bromide group did not show any significant effect. The glycopyrronium hippurate group showed a significant effect only on Day 1 of application. The glycopyrronium salicylate group showed the strongest and significant salivary secretion suppressive effect on all of evaluation days.

[Consideration]

As a result of confirming the salivary secretion suppressive effects by the transdermal absorptive preparations containing various glycopyrronium salts, it was confirmed that the transdermal absorptive preparation comprising glycopyrronium salicylate has the strongest effect. These results indicate that it is possible to provide a transdermal absorptive preparation comprising a glycopyrronium salt exhibiting a high effect on sialorrhea by using glycopyrronium salicylate as an active ingredient.

## Claims

1. A medicine comprising glycopyrronium salicylate or a solvate thereof, wherein the medicine is used for treating or preventing sialorrhea.

2. The medicine according to claim 1, wherein the medicine is used so as to be affixed.

3. The medicine according to claim 2, wherein the medicine is used so as to be affixed once a day.

4. A medicine comprising glycopyrronium salicylate or a solvate thereof, wherein the medicine is used so as to be affixed once a day and be re-affixed every 24 hours for treating or preventing sialorrhea.

5. The medicine according to claims 1 to 4, wherein the medicine is a transdermal absorptive preparation.

6. Glycopyrronium salicylate or a solvate thereof.

7. A medicine comprising glycopyrronium salicylate or a solvate thereof.

8. A transdermal absorptive preparation comprising a plaster layer and a release liner on a backing, wherein the plaster layer comprises an active ingredient and the active ingredient is glycopyrronium salicylate.

[Figure 1]

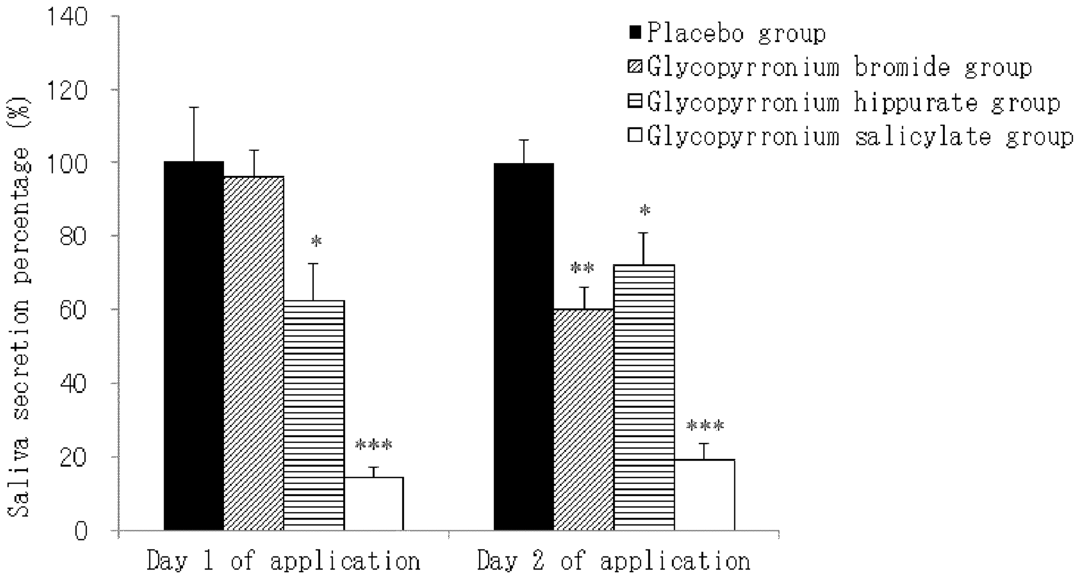


Mean ± S.E.

*:p<0.05, **:p<0.01, ***:p<0.001 : Comparison with placebo group (Dunnett's Test)

[Figure 2]

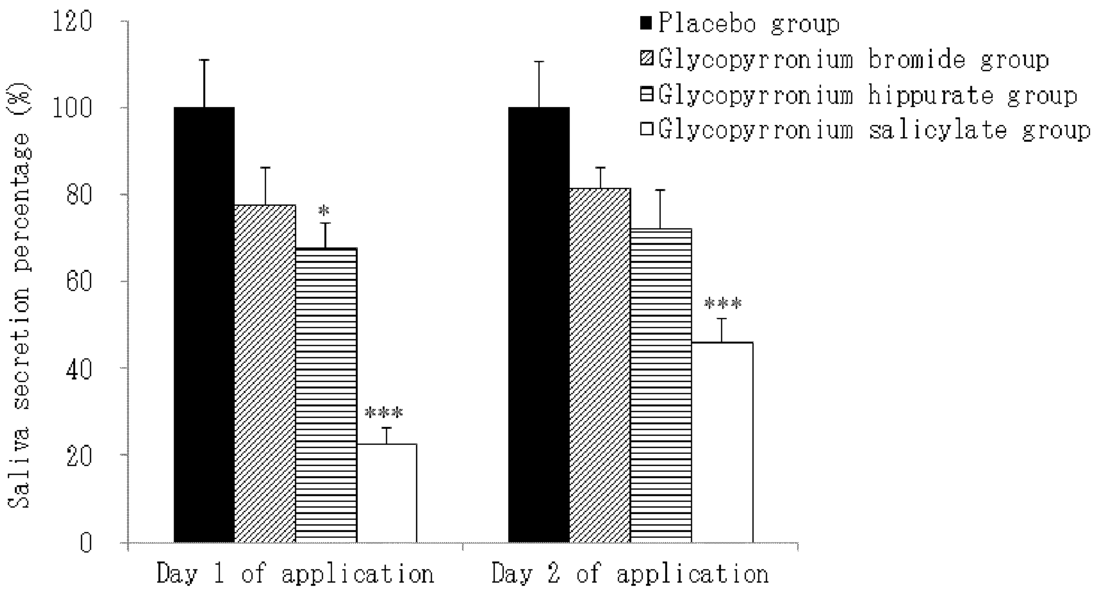


Mean ± S.E.

*:p<0.05, ***:p<0.001 : Comparison with placebo group (Dunnett's Test)

**INTERNATIONAL SEARCH REPORT**

International application No. **PCT/JP2022/004426**

**A. CLASSIFICATION OF SUBJECT MATTER**

***A61K 31/40***(2006.01)i; ***A61K 9/70***(2006.01)i; ***A61P 25/00***(2006.01)i
FI: A61K31/40; A61K9/70 401; A61P25/00

According to International Patent Classification (IPC) or to both national classification and IPC

**B. FIELDS SEARCHED**

Minimum documentation searched (classification system followed by classification symbols)

A61P25/00; A61K9/70; A61K31/40

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | DARWISH, R. A. et al. Optimization of ion-pair formation between glycopyrronium bromide and different ion-pair agents using ACE. Electrophoresis. 2015, vol. 36, pp. 2805-2810<br>abstract, tables 1, 2 | 6-7 |
| A | abstract, tables 1, 2 | 1-5, 8 |
| Y | EILAND, L. S. Glycopyrrolate for Chronic Drooling in Children. Clinical Therapeutics. 2012, vol. 34, no. 4, pp. 735-742<br>abstract, table 1 | 1, 6-7 |
| A | abstract, table 1 | 2-5, 8 |
| Y | WO 2020/020879 A1 (AUGUST WOLFF GMBH & CO. KG ARZNEIMITTEL) 30 January 2020 (2020-01-30)<br>page 5, second paragraph | 1, 6-7 |
| A | page 5, second paragraph | 2-5, 8 |
| A | JP 2010-530902 A (SCIELE PHARMA, INC.) 16 September 2010 (2010-09-16)<br>entire text | 1-8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2022** | **05 April 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/004426**

| Patent document cited in search report | Publication date (day/month/year) | Patent family member(s) | Publication date (day/month/year) |
|---|---|---|---|
| WO 2020/020879 A1 | 30 January 2020 | EP 3826631 A1 | |
| JP 2010-530902 A | 16 September 2010 | US 2008/0317832 A1<br>whole document<br>WO 2009/002935 A1<br>EP 2170305 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008534480 T
- JP 2017128593 A
- JP 2018519289 T
- JP 2010530902 T
- JP 2016510037 T